# EUROPEAN PATENT SPECIFICATION

(11) **EP 3 834 626 B1**
(45) Date of publication and mention of the grant of the patent: **07.10.2026**
(21) Application number: 19847548.5
(22) Date of filing: 08.08.2019
(51) Int. Cl.: A23L 33/00, A61P 1/12, A61K 36/888, A23L 19/00, A23L 33/21, A23L 33/22

(54) **COMPOSITION FOR TREATING DIARRHEA**
ZUSAMMENSETZUNG ZUR BEHANDLUNG VON DIARRHOE
COMPOSITION DE TRAITEMENT DE LA DIARRHÉE

(30) Priority: 10.08.2018 CN 201810909369; 10.08.2018 CN 201810909377
(43) Date of publication of application: 16.06.2021
(73) Proprietor: Cao, Jingjing, Beijing 100000 (CN); Tianjin Geonatural Technology Co., Ltd., Tianjin 301799 (CN)
(72) Inventor: CUI, Weiran, Beijing 100110 (CN); CUI, Yueran, Beijing 100110 (CN); CAO, Jingjing, Beijing 100000 (CN)
(74) Representative: Prinz & Partner mbB
(86) International application number: PCT/CN2019/000155
(87) International publication number: WO 2020/029538

(56) References cited:
- CN-A- 1 895 454
- CN-A- 104 757 420
- CN-A- 105 876 327
- CN-A- 107 361 145
- CN-A- 108 013 453
- CN-A- 108 904 653
- CN-A- 108 904 653
- CN-A- 109 090 456
- CN-A- 109 090 456
- JP-A- 2008 222 822
- US-A1- 2016 192 683
- BAI JIE: "Health diets for gastrointestinal patients", 31 January 2003, CHINA LIGHT INDUSTRY PRESS, CN, ISBN: 978-7-5019-3872-8, article GU KUIQIN: "Paste of lablab purpureus (L) sweet chinese yam and taro", pages: 1 - 2, XP009525753

## Description

### Technical field

The invention relates to a food composition for treating diarrhea, belonging to the food field.

### Background art

Colocasia esculenta, also known as taro or Chinese eddo, is the underground bulb of araceae plants. Its shape and succulent vary from species to variety. The usually eaten species is small taro. Since taro contains a lot of dietary fiber, it also has a good laxative effect.

Some fruits and vegetables are rich in cellulose, which are also conducive to laxative; some fruits and vegetables contain a lot of sugars, which can form a hypertonic environment in the intestinal tract, which will further increase the water in the intestinal tract and aggravate diarrhea.

Cereals are rich in protein, starch and cellulose, and many of them are used for laxatives in daily life.

The above-mentioned raw materials are all daily food materials, and many of them belong to the medicine and food homologous substances. If these common ingredients are used to prepare drugs for treating diarrhea, side effects and adverse reactions will be greatly reduced, which is particularly important for infants and elderly patients.

Most infants and young children are weak or have other problems, and are prone to constipation and diarrhea. Clinically, medications for these groups are very cautious. At present, the clinical treatment of diarrhea for these groups has problems such as few types of drugs and high drug toxicity. The "Chinese Children's Clinical Practice Guidelines for Acute Infectious Diarrhea" clarified that eating early can improve the intestinal osmotic pressure caused by infection, shorten the course of diarrhea, and improve the nutritional status of children; a lactose-free diet can shorten the course of diarrhea in children with acute diarrhea. However, the Guidelines do not clearly indicate which foods to eat during diarrhea in children to help the intestinal recovery. Therefore, it is necessary to develop a safe and effective food for the treatment of infants and young children's diarrhea, through diet therapy, to achieve the purpose of alleviating diarrhea and supplementing nutrition to meet clinical needs.

CN 201810909369.2 discloses a composition for regulating infant diarrhea. The composition has a good antidiarrheal effect on common infant infectious diarrhea, drug-induced diarrhea and food intolerance diarrhea, but has a poor effect on persisting diarrhea caused by severe milk protein allergy, lactose intolerance and inflammatory bowel disease (IBD). The effects of different formulas are quite different. Therefore, it is necessary to further optimize or design a new formula to solve the above problems.

CN 108 904 653 A discloses a pharmaceutical composition and uses thereof, which is composed of taro together with fruits and vegetables in a certain ratio. The composition has corresponding definitions on fruits and vegetables, but because part of the defined fruits and vegetables components are high-fiber plants, and it has been proved by practice that some raw materials have a diarrheal effect on infants and young children. Long-term consumption of the aforementioned foods, such as Chinese cabbage and radishes, during infants and young children's diarrhea, can aggravate diarrhea. Therefore, the components in the composition disclosed in CN 108 904 653 A have flawed technical defect that the antidiarrheal effect is unstable.

CN 109 090 456 A discloses a composition for regulating diarrhea of infants. The composition includes, by weight ratio, 10-90% of dasheen and 10-90% of carrots. The composition is low in cost, raw materials are easy to obtain, and the composition is safe, non-toxic and suitable for industrial production. The product has an obvious antidiarrheal effect and is especially suitable for infants.

CN 1 895 454 A discloses a medicine for treating the acute, metastatic, or chronic diarrhea of baby and the chronic diarrhea of old man, and regulating the intestinal function of baby is prepared from green tea, pot crust, brown sugar, taro, haw and oligose. Its preparing process is also disclosed.

US 2016/192683 A1 discloses are food products and methods for producing the same. In one aspect, the food products comprise one or more root vegetables. Also described are methods for producing food products comprising shredding a root vegetable, exposing the shredded root vegetable to a heated environment for a sufficient time to allow the shredded root vegetable to caramelize and/or dry, cooling the dried root vegetable, and combining the cooled, dried root vegetable with one or more other ingredients, such as seeds, coconut, salt or combinations thereof to form a food product. The food products described herein are substantially free from one or more common food-based allergens (e.g., gluten).

CN 108 013 453 A discloses a special clinical nutritional formula for lactose intolerance in children. The special clinical nutritional formula comprises the following components in parts by weight: 15-22 parts of protein, 6-10 parts of fat, 43-58 parts of carbohydrates, 7-10 parts of dietary fibers, 0.01-0.15 part of major elements, 0.001-0.004 part of trace elements, 0.001-0.003 part of fat-solublevitamins, 0.01-0.03 part of water-soluble vitamins, 0.005-0.02 part of dietary essence, 0.0005-0.05 part of medicinal and edible components, 0.05-0.4 part of a natural plant compound and 0.01-0.02 part of new resource food. The invention also provides preparation methods of two clinical nutritional formulas.

CN 107 361 145 A relates to child black element grain milk. The child black element grain milk is prepared from the following components in parts by mass: 4-8 parts of heterophylly falsestarwort root powder (after removal of root stems and leaf stems), 4-8 parts of poria cocos powder, 4-8 parts of raw bighead atractylodes rhizome powder, 4-8 parts of hyacinth bean powder, 2-6 parts of pitted red jujube powder, 1-2 parts of red rice powder, 1-3 parts of red bean powder, 4-8 parts of lotus seed powder, 4-8 parts of raw coix seed powder, 4-8 parts of raw Chinese yam powder, 4-8 parts of gordon euryale seed powder, 10 parts of rice flour, 3-4 parts of white sugar, 2-3 parts of oat, 30-35 parts of water, and 120-130 parts of fresh milk. Various grains are added to milk, so that when people drink the milk, various grains can be complemented, and the milk is balanced and rich in nutrition and fragrant and mellow in taste.

There is currently no safe and stable food for children on the market. Therefore, it is an urgent problem to develop a safe and effective antidiarrheal diet for infants and young children with safe food material as components.

### Summary of the invention

The object of the present invention is to provide a dietary composition for treating diarrhea. The dietary composition uses common food materials as raw materials to achieve the purpose of antidiarrheal based on reasonable matching, which is safe and does not have side effects, so as to satisfy the need of antidiarrheal of frail people, such as infants and children as well as the patients with radiotherapy and chemotherapy.

Another object of the present invention is to provide a composition for treating diarrhea in infants with milk protein allergy, which can help infants with milk protein allergy to quickly solve the problem of supplementary food.

Another object of the present invention is to provide a composition for the treatment of persistent diarrhea caused by lactose intolerance and milk protein allergy, which can quickly and safely improve the symptoms of chronic diarrhea.

Another object of the present invention is to improve the technical solution disclosed in CN 108 904 653 A, so that the prepared composition can achieve better and more stable antidiarrheal effect.

The object of the invention is solved by the subject matter according to the appended claims, which relate to:
- A composition for treating diarrhea in infants and young children, comprising taro and antidiarrheal food, wherein the antidiarrheal food includes vegetables; the vegetables are carrot; and based on the composition, the ratio of taro by weight is 10-90%, and based on the composition, the ratio of the antidiarrheal food by weight is 10-90% vegetables.
- A method for the preparation of the above composition for treating diarrhea in infants and young children, comprising:
   washing the taro, peeling, steaming, heating for 5 to 30 minutes after the water boils, and drying under 120°C to make powder;
   washing the antidiarrheal food, cutting into pieces or pellets, boiling in water, continue heating for 5 to 30 minutes after the water boils, drying and crushing;
   mixing the taro powder and antidiarrheal food powder according to the ratio, and
   sterilizing to get the composition; preferably, the heating in step is continued for 10 to 15 minutes after the water boils; the drying temperature is less than or equal to 100°C; more preferably, the drying temperature is less than or equal to 80°C; and preferably, the heating in step is continued for 15 to 20 minutes after the water boils.
- A food or medical composition as defined above, for use in the treatment of diarrhea in infants and young children.

In order to solve the above-mentioned problems, there is provided a composition according to claim 1. The components and ratio by weight of the pharmaceutical composition of the present invention comprise the taro and antidiarrheal food in combination to feed children with diarrhea. The combination is operated according to ratio by weight.

In the process of many years of dietary treatment of infants and children with diarrhea, the inventor has conducted continuous observations on the every meal feeding and daily excretion of more than 2000 children with diarrhea, especially those with milk protein allergy, from 2 months to 18 months. And after analyzing millions of pieces of data, the inventor found that some common foods have the function of restraining and stopping diarrhea in children's intestines. The antidiarrheal food can be made of fresh raw materials in a ratio by weight, or can be made into dry powder and then formulated.

In the composition of the present invention, the antidiarrheal food includes carrot and one or more selected from the group consisting of rice and glutinous rice.

Preferably, the taro is taro powder or taro puree; the antidiarrheal food is in a dry powder or pureed form.

The antidiarrheal foods of the present invention also comprises foods with astringent and antidiarrheal effects recorded in classical Chinese medicine books, such as "Huangdi Neijing", "Compendium of Materia Medica", "Shen Nong's Materia Medica", or other medicine and food homologous substances.

The components of the composition comprise powder, puree and granule; preferably powder. Powder of the raw material can be purchased from the open market or self-made.

The invention also discloses a method for the preparation of the composition, comprising:
(1) washing the taro, peeling, steaming, heating for 5 to 30 minutes after the water boils, and drying under 120°C to make powder;
(2) washing the antidiarrheal food, cutting into pieces or pellets, boiling in water, continually heating for 5 to 30 minutes after the water boils, drying and crushing;
(3) mixing the taro powder and antidiarrheal food powder according to the ratio, and sterilizing to get the composition.

Preferably, the heating in step (1) is continued for 10 to 15 minutes after the water boils; the drying temperature is ≤100°C; more preferably, the drying temperature is ≤80°C.

Preferably, the heating in step (2) is continued for 15 to 20 minutes after the water boils.

As the antidiarrheal food in the composition, commercially available ones can be selected.

When preparing the composition of the present invention, the drying method comprises roller drying, freeze drying and air drying.

The composition of the invention is suitable for viral diarrhea, bacterial diarrhea, allergic diarrhea, lactose intolerant diarrhea, drug-induced diarrhea, congenital megacolon diarrhea or inflammatory bowel disease (IBD) diarrhea.

1 to 7 grams each time for infants from 6 months to 36 months, 1 to 6 times a day; and 2 to 10 grams each time for children over 36 months old, 1 to 3 times a day.

The composition of the present invention has low cost, available raw materials, is safe and non-toxic, and is suitable for industrial production. The antidiarrheal effect of the product is remarkable. The rehabilitation time for common diarrhea is 24 to 48 hours, and for the diarrhea caused by rotavirus is 48 to 72 hours, and the rehabilitation time is shortened by 2.5 days on average. The effective rate for persistent diarrhea within 5 days is 90.16%. For diarrhea of infants with milk protein allergy during the addition of supplementary feeding, six to eight kinds of foods can be added in one month, and thus the composition is especially suitable for infants and young children.

### Best mode of the invention

The components and ratio by weight of the composition of the present invention comprise the taro and antidiarrheal food in combination to feed children with diarrhea. The combination can be operated according to ratio by weight. It can be confirmed that the composition of the present invention has a good antidiarrheal effect within the above-mentioned ratio. The following examples are merely illustrative, and do not intent to limit the protection scope of the present invention.

In the first embodiment of the present invention, taking 100kg of taro and antidiarrheal food respectively, washing the taro, peeling, steaming, heating for 5 to 30 minutes after the water boils, and drying under 120°C to make powder; washing the antidiarrheal food, cutting into pieces or pellets, boiling in water, continually heating for 5 to 30 minutes after the water boils, drying and crushing; mixing the taro powder and antidiarrheal food powder according to the ratio, and sterilizing to get the composition.

Preferably, when preparing the taro powder, heating is continued for 10 to 15 minutes after the water boils, and the drying temperature is ≤100°C; more preferably, the drying temperature is ≤80°C.

Preferably, when preparing the antidiarrheal food powder, heating is continued for 15 to 20 minutes after the water boils.

In the second embodiment of the present invention, the taro powder and antidiarrheal food powder prepared in Example 1 are mixed according to the ratio by weight to prepare the composition of the present invention. Preferably, the taro and the antidiarrheal food are formulated according to the following ratio by weight: 25-75% taro and 25-75% antidiarrheal food; further preferably, 30-70% taro and 30-70% antidiarrheal food; further preferably, 35-65% taro and 35-65% antidiarrheal food; further preferably, 40-60% taro and 40-60% antidiarrheal food; more preferably, 50% taro and 50% antidiarrheal food.

Within the above-mentioned ratio ranges, the ratio of taro and antidiarrheal food by weight may be 75% taro and 25% antidiarrheal food; 70% taro and 30% antidiarrheal food; 65% taro and 35% antidiarrheal food; 60% taro and 40% antidiarrheal food; 55% taro and 45% antidiarrheal food; 45% taro and 55% antidiarrheal food; 40% taro and 60% antidiarrheal food, 35% taro and 65% antidiarrheal food; 30% taro and 70% antidiarrheal food; 25% taro and 75% antidiarrheal food. The ratio of the taro and the antidiarrheal food by weight can also be any ratio between the weight percentages of the above groups, such as 60-65% taro and 35-40% antidiarrheal food; 33-64% taro and 36-67% antidiarrheal food, etc.

Preferably, the taro is taro puree; the antidiarrheal food is in a pureed form. The antidiarrheal food of the present invention includes vegetables and optionally cereals. According to the present invention, the vegetables are carrot.

The cereals include one or more selected from the group consisting of rice and glutinous rice, and may further include sorghum rice.

The antidiarrheal food of the present invention can be one or more combinations in any ratio by weight.

Preferably, the antidiarrheal food includes carrot and cereals selected from the group consisting of rice and glutinous rice, and may further include one or more selected from the group consisting of purple sweet potato, beef, chestnuts, jujube, small red bean, azuki bean, and sorghum rice.

More preferably, the antidiarrheal food includes carrot and cereals selected from the group consisting of rice and glutinous rice, and may further include one or more selected from the group consisting of purple sweet potato, jujube, small red bean, azuki bean, and sorghum rice.

Further preferably, the antidiarrheal food includes carrot and cereals selected from the group consisting of rice and glutinous rice, and may further include one or more selected from the group consisting of purple sweet potato, jujube, and sorghum rice.

More preferably, the antidiarrheal food includes carrot and cereals selected from the group consisting of rice and glutinous rice, and may further include one or more selected from the group consisting of jujube, and sorghum rice.

Furthermore preferably, the antidiarrheal food includes carrot, and cereals selected from the group consisting of rice and glutinous rice.

The raw materials in each component in this embodiment can be combined arbitrarily, and each component can be formulated within a limited range, and the final prepared composition has the same antidiarrheal effect.

In the third embodiment of the present invention, the taro powder and antidiarrheal food powder prepared in Example 1 are mixed according to the ratio by weight to prepare the composition of the present invention.

The fruits or nuts include one or more selected from the group consisting of cooked apple and jujube.

The beans include small red bean and/or azuki bean.

The cereals include one or more selected from the group consisting of rice and glutinous rice, and may further include sorghum rice.

According to an embodiment of the present invention, the antidiarrheal food is made of vegetables and cereals in a ratio by weight. The ratio of the antidiarrheal food by weight comprises the followings:
10-90% vegetables and 10-90% cereals.
Preferably, 25-75% vegetables and 25-75% cereals.
More preferably, 30-70% vegetables and 30-70% cereals.
Further preferably, 40-60% vegetables and 40-60% cereals.
Furthermore preferably, 50% vegetables and 50% cereals.

In the composition of the present invention, the vegetables are carrot.

In a further embodiment of the present invention, in order to meet the need of growth and development of infants and young children, the trace elements and minerals can be added to the composition of the present invention to prepare a complete nutritional formula.

Preferably, the energy per 100g of the composition is ≥1250kJ; the nutrients per 100kJ include:
protein ≥0.33g, fat ≤0.8g, vitamin A 14-43µgRE, vitamin D 0.25-0.75µg, vitamin B1 ≥12.5µg, calcium ≥12mg, iron 0.25-0.5mg, zinc ≥0.17-0.46mg, and sodium ≤24mg;
the following contents of vitamins and minerals can also be added to the composition:
   vitamin E 0.08-1.2mg, vitamin B2 ≥13µg, vitamin B6 ≥8.4µg, vitamin B12 ≥0.02µg, niacin ≥83.7µg, folic acid ≥1.2µg, pantothenic acid ≥50.4µg, vitamin C ≥1.4mg, biotin ≥0.17µg, phosphorus 8.4-30mg, iodine 1.4-8.8µg, and potassium 13-66mg.

More preferably, in order to further meet the need of growth and development of infants under 1 year old during diarrhea, the nutrients and contents contained per 100kJ in the composition of the present invention comprise: protein 0.45-0.7g, fat 1.05-1.4g, carbohydrate 2.2-3.3g, vitamin A 14-43µgRE, vitamin D 0.25-0.6µg, vitamin E 0.12-1.2mg, vitamin K1 1.0-6.5µg, vitamin B1 14-72µg, vitamin B2 19-119µg, vitamin B6 8.5-45µg, vitamin B12 0.02-0.36µg, niacin 70-360µg, folic acid 2.5-12µg, pantothenic acid 96-478µg, vitamin C 2.5-17mg, biotin 0.4-2.4µg, sodium 5-14mg, potassium 14-43mg, copper 8.5-29µg, magnesium 1.2-3.6mg, iron 0.1-0.36mg, zinc 0.12-0.36mg, manganese 1.2-24µg, calcium 12-35mg, phosphorus 6-24mg, the ratio of calcium to phosphorus 1:1-2:1, iodine 2.5-14µg, chlorine 12-38mg, and selenium 0.48-1.9µg;
preferably, the following contents of trace elements can also be added to the composition:
chromium 0.4-2.4µg, molybdenum 0.4-2.4µg, choline 1.7-12mg, inositol 1-9.5mg, taurine ≤3mg, L-carnitine ≥0.3mg, docosahexaenoic acid (% of total fatty acids) ≤0.5, and eicosatetraenoic acid (% of total fatty acids) ≤1.

Further preferably, in order to further meet the need of growth and development of children over 1 year old during diarrhea, the nutrients and content contained per 100kJ in the composition of the present invention include: vitamin A 17.9-58.3µgRE, vitamin D 0.25-0.75µg, vitamin E ≥0.15mg, vitamin K1 ≥1µg, vitamin B1 ≥0.01mg, vitamin B2 ≥0.01mg, vitamin B6 ≥0.01mg, vitamin B12 ≥0.04µg, niacin ≥0.11 mg, folic acid ≥1µg, pantothenic acid ≥0.07mg, vitamin C ≥1.8mg, biotin ≥0.4 µg, sodium 5-20mg, potassium 18-69mg, copper 7-35µg, magnesium ≥1.4mg, iron 0.25-0.5mg, zinc 0.1-0.4mg, manganese 0.3-24µg, calcium ≥17mg, phosphorus 8.3-46.2mg, the ratio of calcium to phosphorus 1:1-2:1, iodine ≥1.4µg, chlorine ≤52mg, and selenium 0.5-2.9µg;
further preferably, the following content of trace elements can also be added to the composition: chromium 0.4-5.7µg, molybdenum 1.2-5.7µg, fluorine ≤0.05mg, choline 1.7-19.1mg, inositol 1-9.5mg, taurine ≤3.1mg, L-carnitine ≥0.3mg, docosahexaenoic acid (% of total fatty acids) ≤ 0.5, eicosatetraenoic acid (% of total fatty acids) ≤ 1, nucleotides ≥ 0.5 mg, and dietary fiber ≤ 0.7 g.

The components of the composition comprise powder, puree form and granule; preferably powder. The powder of raw material can be purchased from the open market or self-made.

3.5 to 7 grams each time for infants from 6 months to 3 years old, 2 to 3 times a day; and 5 to 10 grams each time for children over 36 months old, 2 to 3 times a day.

The composition of the present invention is suitable for the treatment and nutritional support of viral diarrhea, bacterial diarrhea, allergic diarrhea, lactose intolerant diarrhea, drug-induced diarrhea, congenital megacolon diarrhea or inflammatory bowel disease (IBD) diarrhea.

The pharmaceutical composition of the present invention has obvious antidiarrheal function. Those skilled in the art can change the components through equivalent substitutions, etc., but all fall within the protection scope of the present invention.

### Test example

### Experimental example 1. Clinical observation of the composition of the present invention in treating viral diarrhea

### 1. Clinical data

1.1 General information. 108 children outpatients with diarrhea were randomly divided into treatment group (54 cases) and control group (54 cases). All cases had obvious dehydration before treatment, and the number of diarrhea in 24 hours was more than 5 times. There were 54 cases in the treatment group, including 31 boys and 23 girls, aged 1 to 5 years old, with an average of 2.7 years old. There were 54 cases in the control group, including 34 boys and 20 girls, aged from 1 to 4.8 years old, with an average of 2.4 years old. After statistical processing, the clinical data of the two groups are comparable (P>0.05).

1.2 Treatment methods. The two groups were given symptomatic treatment such as rehydration. The treatment group orally took the composition prepared in Example 2 of the present invention with 50% taro, 25% carrot, 25% rice and glutinous rice in the ratio by weight. For infants with diarrhea from 6 months to 3 years old, 3.5 grams of the pharmaceutical composition were rinsed with warm water and taken orally, 6 times a day. For children with diarrhea over 3 years old, 7 grams the pharmaceutical composition were rinsed with warm water and taken orally, 3 times a day. Taking for 3 consecutive days is one course of treatment. The frequency and severity of diarrhea were observed, and routine blood tests, urine tests, liver function tests and kidney function tests were carried out before and after treatment. The control group took the eight-sided montmorillonite powder orally according to the method and dosage of the instructions.

1.3 The standardization of efficacy evaluation is based on "Diagnostic Criteria and Principles of Treatment of Infectious Diarrhea". Significantly effective: diarrhea frequency in 24h ≤ 2 times; Effective: 3 times ≤ diarrhea frequency in 24h ≤ 5 times; and Ineffective: diarrhea frequency in 24h > 5 times, the diarrhea frequency is not significantly reduced or excreting watery stools.

### 2. Results

2.1 Comparison of the treatment effect for diarrhea between the two groups is shown in Table 1.

**Table 1. Comparison of the treatment effect for diarrhea between the two groups**

| Groups | Cases | Significantly effective | | Effective | | Ineffective | | Total effective rate |
|---|---|---|---|---|---|---|---|---|
| | | n | % | n | % | n | % | % |
| Treatment group | 54 | 49 | 90.74 | 4 | 7.41 | 1 | 1.85 | 98.15 |
| Control group | 54 | 43 | 79.63 | 6 | 11.11 | 5 | 9.26 | 90.74 |

### 3. Discussion

Both the composition of the present invention and the control group have the effects of treating diarrhea and constricting the intestinal tract. The effect of the composition group of the invention is better than that of the control group.

### Experimental example 2.

Clinical observation of the pharmaceutical composition of the present invention in treating diarrhea caused by lactose intolerance and milk protein allergy diarrhea in infants and children.

### 1. Clinical data

1.1 General information. 100 outpatients with persistent diarrhea caused by lactose intolerance and milk protein allergy were randomly divided into treatment group (50 cases) and control group (50 cases). There were 80 children with lactose intolerance and persistent diarrhea, 60 children with milk protein allergy, and 40 children with milk protein allergy and lactose intolerance. All cases had symptoms of diarrhea after consuming breast milk or hydrolyzed formula powder containing lactose before treatment. Diarrhea occurred half an hour after breastfeeding and was detected as lactose intolerance and/or milk protein allergy. There were 50 cases in the treatment group, including 35 boys and 15 girls, aged from 6 months to 3 years old, with an average of 1.6 years old. There were 50 cases in the control group, including 26 boys and 24 girls, aged from 7 months to 2.5 years old, with an average age of 1.4 years old. After statistical processing, the clinical data of the two groups are comparable (P>0.05).

1.2 Therapeutic method. The treatment group orally took the composition prepared in Example 2 of the present invention with 25% taro, 20% carrot, 30% rice and 25% glutinous rice in the ratio by weight. For infants with diarrhea from 6 months to 3 years old, 3.5 grams of the pharmaceutical composition were rinsed with warm water and taken orally, 6 times a day. For children with diarrhea over 3 years old, 7 grams the pharmaceutical composition were rinsed with warm water and taken orally, 3 times a day. Taking for 5 consecutive days is one course of treatment. The frequency of diarrhea was observed before and after treatment. The control group orally took lactase according to the method and dosage of the instructions.

1.3 The standardization of efficacy evaluation. Significantly effective: diarrhea frequency in 4 hours after feeding ≤ 1 time; Effective: 1 time < diarrhea frequency in 4 hours after feeding ≤ 2 times; and Ineffective: diarrhea frequency in 4 hours after feeding ≥ 3 times, the diarrhea frequency is not significantly reduced or there is no obvious reduction in watery stools.

### 2. Results

2.1 Comparison of the treatment effect for diarrhea between the two groups is shown in Table 2.

**Table 2. Comparison of the treatment effect for diarrhea between the two groups**

| Groups | Cases | Significantly effective | | Effective | | Ineffective | | Total effective rate |
|---|---|---|---|---|---|---|---|---|
| | | n | % | n | % | n | % | % |
| Treatment group | 50 | 37 | 74.00 | 11 | 22.00 | 2 | 4.00 | 96.00 |
| Control group | 50 | 28 | 56.00 | 13 | 26.00 | 9 | 18.00 | 82.00 |

### 3. Discussion

Both the composition of the present invention and the control group have the effects of treating diarrhea caused by lactose intolerance and milk protein allergy and constricting the intestinal tract. The effect of the composition group of the invention is better than that of the control group.

## Claims

1. A composition for treating diarrhea in infants and young children, comprising taro and antidiarrheal food, wherein the antidiarrheal food includes vegetables;
the vegetables are carrot; and
based on the composition, the ratio of taro by weight is 10-90%, and based on the composition, the ratio of the antidiarrheal food by weight is 10-90% vegetables.

2. The composition according to claim 1, **characterized in that** based on the composition, the ratio of taro by weight is 40-60%, and based on the composition the ratio of carrot by weight is 40-60%.

3. The composition according to claim 1, **characterized in that** based on the composition, the ratio of taro by weight is 50%, and based on the composition the ratio of antidiarrheal food by weight is 50%, wherein the ratio of the antidiarrheal food by weight comprises 50% vegetables and 50% cereals.

4. The composition according to claim 3, **characterized in that** the cereals include one or more selected from the group consisting of rice and glutinous rice.

5. The composition according to any one of claims 1 to 4, **characterized in that** the trace elements with following contents are added to the composition:
the energy per 100g of the composition is ≥1250kJ; the nutrients per 100kJ include: protein ≥0.33g, fat ≤0.8g, vitamin A 14-43µgRE, vitamin D 0.25-0.75µg, vitamin B1 ≥12.5µg, calcium ≥12mg, iron 0.25-0.5mg, zinc ≥0.17-0.46mg, and sodium ≤24mg;
more preferably, the following contents of vitamins and minerals are added to the composition:
vitamin E 0.08-1.2mg, vitamin B2 ≥13µg, vitamin B6 ≥8.4µg, vitamin B12 ≥0.02µg, niacin ≥83.7µg, folic acid ≥1.2µg, pantothenic acid ≥50.4µg, vitamin C ≥1.4mg, biotin ≥0.17µg, phosphorus 8.4-30mg, iodine 1.4-8.8µg, and potassium 13-66mg.

6. The composition according to any one of claims 1 to 4, **characterized in that** the nutrients and contents contained per 100kJ in the composition comprise: protein 0.45-0.7g, fat 1.05-1.4g, carbohydrate 2.2-3.3g, vitamin A 14-43µgRE, vitamin D 0.25-0.6µg, vitamin E 0.12-1.2mg, vitamin K1 1.0-6.5µg, vitamin B1 14-72µg, vitamin B2 19-119µg, vitamin B6 8.5-45µg, vitamin B12 0.02-0.36µg, niacin 70-360µg, folic acid 2.5-12µg, pantothenic acid 96-478µg, vitamin C 2.5-17mg, biotin 0.4-2.4µg, sodium 5-14mg, potassium 14-43mg, copper 8.5-29µg, magnesium 1.2-3.6mg, iron 0.1-0.36mg, zinc 0.12-0.36mg, manganese 1.2-24µg, calcium 12-35mg, phosphorus 6-24mg, the ratio of calcium to phosphorus 1:1-2:1, iodine 2.5-14µg, chlorine 12-38mg, and selenium 0.48-1.9µg.

7. The composition according to any one of claims 1 to 4, **characterized in that** the composition further comprises any one or more of the following content of trace elements:
chromium 0.4-2.4µg, molybdenum 0.4-2.4µg, choline 1.7-12mg, inositol 1-9.5mg, taurine ≤3mg, L-carnitine≥0.3mg, docosahexaenoic acid (% of total fatty acids) ≤0.5, and eicosatetraenoic acid (% of total fatty acids) ≤1.

8. The composition according to any one of claims 1 to 4, **characterized in that** the nutrients and content contained per 100kJ in the composition include:
vitamin A 17.9-58.3µgRE, vitamin D 0.25-0.75µg, vitamin E ≥0.15mg, vitamin K1 ≥1µg, vitamin B1 ≥0.01mg, vitamin B2 ≥0.01mg, vitamin B6 ≥0.01mg, vitamin B12 ≥0.04µg, niacin ≥0.11mg, folic acid ≥1µg, pantothenic acid ≥0.07mg, vitamin C ≥1.8mg, biotin ≥0.4 µg, sodium 5-20mg, potassium 18-69mg, copper 7-35µg, magnesium ≥1.4mg, iron 0.25-0.5 mg, zinc 0.1-0.4mg, manganese 0.3-24µg, calcium ≥17mg, phosphorus 8.3-46.2mg, the ratio of calcium to phosphorus 1:1-2:1, iodine ≥1.4µg, chlorine ≤52mg, and selenium 0.5-2.9µg;
preferably, the following contents of trace elements are further added to the composition: chromium 0.4-5.7µg, molybdenum 1.2-5.7µg, fluorine ≤0.05mg, choline 1.7-19.1mg, inositol 1-9.5mg, taurine ≤3.1mg, L-carnitine ≥0.3mg, docosahexaenoic acid (% of total fatty acids) ≤ 0.5, eicosatetraenoic acid (% of total fatty acids) ≤ 1, nucleotides ≥ 0.5 mg, and dietary fiber ≤ 0.7 g.

9. The composition according to any one of claims 1 to 4, **characterized in that** the composition is powder.

10. A method for the preparation of the composition for treating diarrhea in infants and young children according to any one of claims 1 to 9, comprising:
(1) washing the taro, peeling, steaming, heating for 5 to 30 minutes after the water boils, and drying under 120°C to make powder;
(2) washing the antidiarrheal food, cutting into pieces or pellets, boiling in water, continue heating for 5 to 30 minutes after the water boils, drying and crushing;
(3) mixing the taro powder and antidiarrheal food powder according to the ratio, and sterilizing to get the composition;
preferably, the heating in step (1) is continued for 10 to 15 minutes after the water boils; the drying temperature is less than or equal to 100°C; more preferably, the drying temperature is less than or equal to 80°C; and
preferably, the heating in step (2) is continued for 15 to 20 minutes after the water boils.

11. A food or medical composition according to any one of claims 1 to 9, for use in the treatment of diarrhea in infants and young children.

12. The food or medicine composition according to claim 11, for use in the treatment of diarrhea in infants and young children, wherein the diarrhea includes viral diarrhea, bacterial diarrhea, allergic diarrhea, lactose intolerant diarrhea, drug-induced diarrhea and diarrhea caused by other reasons;
preferably, wherein the composition is administered as follows: 1 to 7 grams each time for infants from 6 months to 36 months, 1 to 6 times a day; and 2 to 10 grams each time for children over 36 months old, 1 to 3 times a day.

## Patentansprüche

1. Zusammensetzung zur Behandlung von Diarrhoe bei Säuglingen und Kleinkindern, die Taro und antidiarrhoische Nahrungsmittel umfasst, wobei die antidiarrhoischen Nahrungsmittel Gemüse enthalten,
es sich bei dem Gemüse um Karotte handelt und
der Gewichtsanteil an Taro bezogen auf die Zusammensetzung 10-90 % beträgt und der Gewichtsanteil der antidiarrhoischen Nahrungsmittel bezogen auf die Zusammensetzung 10-90 % Gemüse beträgt.

2. Zusammensetzung nach Anspruch 1, **dadurch gekennzeichnet, dass** der Gewichtsanteil an Taro bezogen auf die Zusammensetzung 40-60 % beträgt und der Gewichtsanteil an Karotte bezogen auf die Zusammensetzung 40-60 % beträgt.

3. Zusammensetzung nach Anspruch 1, **dadurch gekennzeichnet, dass** der Gewichtsanteil an Taro bezogen auf die Zusammensetzung 50 % beträgt und der Gewichtsanteil an antidiarrhoischen Nahrungsmitteln bezogen auf die Zusammensetzung 50 % beträgt, wobei der Gewichtsanteil der antidiarrhoischen Nahrungsmittel 50 % Gemüse und 50 % Cerealien umfasst.

4. Zusammensetzung nach Anspruch 3, **dadurch gekennzeichnet, dass** zu den Cerealien eine oder mehrere gehören, die aus der aus Reis und Klebreis bestehenden Gruppe ausgewählt sind.

5. Zusammensetzung nach einem der Ansprüche 1 bis 4, **dadurch gekennzeichnet, dass** der Zusammensetzung die Spurenelemente mit folgendem Gehalt zugesetzt sind:
die Energie pro 100 g der Zusammensetzung beträgt ≥ 1250 kJ; die Nährstoffe pro 100 kJ enthalten Folgendes: Eiweiß ≥ 0,33 g, Fett ≤ 0,8 g, Vitamin A 14-43 µg RE, Vitamin D 0,25-0,75 µg, Vitamin B1 ≥ 12,5 µg, Calcium ≥ 12 mg, Eisen 0,25-0,5 mg, Zink ≥ 0,17-0,46 mg und Natrium ≤ 24 mg,
wobei weiter bevorzugt der Zusammensetzung der folgende Gehalt an Vitaminen und Mineralstoffen zugesetzt ist:
Vitamin E 0,08-1,2 mg, Vitamin B2 ≥ 13 µg, Vitamin B6 ≥ 8,4 µg, Vitamin B12 ≥ 0,02 µg, Niacin ≥ 83,7 µg, Folsäure ≥ 1,2 µg, Pantothensäure ≥ 50,4 µg, Vitamin C ≥ 1,4 mg, Biotin ≥ 0,17 µg, Phosphor 8,4-30 mg, Jod 1,4-8,8 µg und Kalium 13-66 mg.

6. Zusammensetzung nach einem der Ansprüche 1 bis 4, **dadurch gekennzeichnet, dass** die in der Zusammensetzung pro 100 kJ enthaltenen Nährstoffe und Gehalte Folgendes umfassen: Eiweiß 0,45-0,7 g, Fett 1,05-1,4 g, Kohlenhydrat 2,2-3,3 g, Vitamin A 14-43 µg RE, Vitamin D 0,25-0,6 µg, Vitamin E 0,12-1,2 mg, Vitamin K1 1,0-6,5 µg, Vitamin B1 14-72 µg, Vitamin B2 19-119 µg, Vitamin B6 8,5-45 µg, Vitamin B12 0,02-0,36 µg, Niacin 70-360 µg, Folsäure 2,5-12 µg, Pantothensäure 96-478 µg, Vitamin C 2,5-17 mg, Biotin 0,4-2,4 µg, Natrium 5-14 mg, Kalium 14-43 mg, Kupfer 8,5-29 µg, Magnesium 1,2-3,6 mg, Eisen 0,1-0,36 mg, Zink 0,12-0,36 mg, Mangan 1,2-24 µg, Calcium 12-35 mg, Phosphor 6-24 mg, wobei das Verhältnis von Calcium zu Phosphor 1:1-2:1 beträgt, Jod 2,5-14 µg, Chlor 12-38 mg und Selen 0,48-1,9 µg.

7. Zusammensetzung nach einem der Ansprüche 1 bis 4, **dadurch gekennzeichnet, dass** die Zusammensetzung ferner ein oder mehrere Spurenelemente mit folgendem Gehalt umfasst:
Chrom 0,4-2,4 µg, Molybdän 0,4-2,4 µg, Cholin 1,7-12 mg, Inositol 1-9,5 mg, Taurin ≤ 3 mg, L-Carnitin ≥ 0,3 mg, Docosahexaensäure (in % der gesamten Fettsäuren) ≤ 0,5 und Eicosatetraensäure (in % der gesamten Fettsäuren) ≤ 1.

8. Zusammensetzung nach einem der Ansprüche 1 bis 4, **dadurch gekennzeichnet, dass** die in der Zusammensetzung pro 100 kJ enthaltenen Nährstoffe und der Gehalt Folgendes umfassen: Vitamin A 17,9-58,3 µg RE, Vitamin D 0,25-0,75 µg, Vitamin E ≥ 0,15 mg, Vitamin K1 ≥ 1 µg, Vitamin B1 ≥ 0,01 mg, Vitamin B2 ≥ 0,01 mg, Vitamin B6 ≥ 0,01 mg, Vitamin B12 ≥ 0,04 µg, Niacin ≥ 0,11 mg, Folsäure ≥ 1 µg, Pantothensäure ≥ 0,07 mg, Vitamin C ≥ 1,8 mg, Biotin ≥ 0,4 µg, Natrium 5-20 mg, Kalium 18-69 mg, Kupfer 7-35 µg, Magnesium ≥ 1,4 mg, Eisen 0,25-0,5 mg, Zink 0,1-0,4 mg, Mangan 0,3-24 µg, Calcium ≥ 17 mg, Phosphor 8,3-46,2 mg, wobei das Verhältnis von Calcium zu Phosphor 1:1-2:1 beträgt, Jod ≥ 1,4 µg, Chlor ≤ 52 mg und Selen 0,5-2,9 µg,
wobei vorzugsweise der Zusammensetzung ferner der folgende Gehalt an Spurenelementen zugesetzt ist: Chrom 0,4-5,7 µg, Molybdän 1,2-5,7 µg, Fluor ≤ 0,05 mg, Cholin 1,7-19,1 mg, Inositol 1-9,5 mg, Taurin ≤ 3,1 mg, L-Carnitin ≥ 0,3 mg, Docosahexaensäure (in % der gesamten Fettsäuren) ≤ 0,5, Eicosatetraensäure (in % der gesamten Fettsäuren) ≤ 1, Nukleotide ≥ 0,5 mg und Ballaststoffe ≤ 0,7 g.

9. Zusammensetzung nach einem der Ansprüche 1 bis 4, **dadurch gekennzeichnet, dass** die Zusammensetzung pulverförmig ist.

10. Verfahren zur Herstellung der Zusammensetzung zur Behandlung von Diarrhoe bei Säuglingen und Kleinkindern nach einem der Ansprüche 1 bis 9, das Folgendes umfasst:
(1) Waschen des Taros, Schälen, Dämpfen, nach dem Sieden des Wassers 5 bis 30 Minuten lang erhitzen und bei unter 120°C trocknen, um Pulver herzustellen,
(2) Waschen des antidiarrhoischen Nahrungsmittels, Schneiden in Stücke oder Pellets, Kochen in Wasser, Fortsetzen des Erhitzens 5 bis 30 Minuten lang nach dem Sieden des Wassers, Trocknen und Zerkleinern,
(3) Mischen des Taropulvers und des Pulvers aus dem antidiarrhoischen Nahrungsmittel entsprechend dem Verhältnis und Sterilisieren zur Gewinnung der Zusammensetzung,
wobei vorzugsweise das Erhitzen in Schritt (1) nach dem Sieden des Wassers 10 bis 15 Minuten lang fortgesetzt wird, die Trocknungstemperatur 100 °C oder weniger beträgt, weiter bevorzugt die Trocknungstemperatur 80 °C oder weniger beträgt, und
vorzugsweise das Erhitzen in Schritt (2) nach dem Sieden des Wassers 15 bis 20 Minuten lang fortgesetzt wird.

11. Nahrungsmittel- oder medizinische Zusammensetzung nach einem der Ansprüche 1 bis 9 zur Verwendung bei der Behandlung von Diarrhoe bei Säuglingen und Kleinkindern.

12. Nahrungsmittel- oder medizinische Zusammensetzung nach Anspruch 11 zur Verwendung bei der Behandlung von Diarrhoe bei Säuglingen und Kleinkindern, wobei zu der Diarrhoe virale Diarrhoe, bakterielle Diarrhoe, allergische Diarrhoe, durch Laktoseintoleranz verursachte Diarrhoe, arzneimittelbedingte Diarrhoe und durch andere Ursachen hervorgerufene Diarrhoe gehören,
vorzugsweise wobei die Zusammensetzung wie folgt verabreicht wird: jeweils 1 bis 7 Gramm für Säuglinge von 6 Monaten bis 36 Monaten, 1 bis 6 Mal täglich, und jeweils 2 bis 10 Gramm für Kinder über 36 Monate, 1 bis 3 Mal täglich.

## Revendications

1. Composition pour le traitement de la diarrhée chez les nourrissons et les jeunes enfants, comprenant du taro et un aliment antidiarrhéique, l'aliment antidiarrhéique comprenant des légumes,
les légumes étant des carottes, et
le rapport en poids de taro étant de 10 à 90 % par rapport à la composition, et le rapport en poids de l'aliment antidiarrhéique étant de 10 à 90 % de légumes par rapport à la composition.

2. Composition selon la revendication 1, **caractérisée en ce que**, par rapport à la composition, le rapport en poids de taro est de 40 à 60 %, et, par rapport à la composition, le rapport en poids de carottes est de 40 à 60 %.

3. Composition selon la revendication 1, **caractérisée en ce que**, par rapport à la composition, le rapport en poids de taro est de 50 %, et, par rapport à la composition, le rapport en poids de l'aliment antidiarrhéique est de 50 %, le rapport en poids de l'aliment antidiarrhéique comprenant 50 % de légumes et 50 % de céréales.

4. Composition selon la revendication 3, **caractérisée en ce que** les céréales comprennent une ou plusieurs céréales choisies dans le groupe constitué de riz et de riz gluant.

5. Composition selon l'une des revendications 1 à 4, **caractérisée en ce que** les oligo-éléments dont les teneurs sont indiquées ci-après sont ajoutés à la composition :
l'énergie pour 100 g de composition est ≥ 1250 kJ ; les nutriments pour 100 kJ comprennent : protéines ≥ 0,33 g, lipides ≤ 0,8 g, vitamine A 14 à 43 µgRE, vitamine D 0,25 à 0,75 µg, vitamine B1 ≥ 12,5 µg, calcium ≥ 12 mg, fer 0,25 à 0,5 mg, zinc ≥ 0,17 à 0,46 mg et sodium ≤ 24 mg ;
les teneurs suivantes en vitamines et minéraux étant de préférence ajoutées à la composition :
vitamine E 0,08 à 1,2 mg, vitamine B2 ≥ 13 µg, vitamine B6 ≥ 8,4 µg, vitamine B12 ≥ 0,02 µg, niacine ≥ 83,7 µg, acide folique ≥ 1,2 µg, acide pantothénique ≥ 50,4 µg, vitamine C ≥ 1,4 mg, biotine ≥ 0,17 µg, phosphore 8,4 à 30 mg, iode 1,4 à 8,8 µg et potassium 13 à 66 mg

6. Composition selon l'une des revendications 1 à 4, **caractérisée en ce que** les nutriments et leurs teneurs contenues pour 100 kJ dans la composition comprennent : protéines 0,45 à 0,7 g, lipides 1,05 à 1,4 g, glucides 2,2 à 3,3 g, vitamine A 14 à 43 µgRE, vitamine D 0,25 à 0,6 µg, vitamine E 0,12 à 1,2 mg, vitamine K1 1,0 à 6,5 µg, vitamine B1 14 à 72 µg, vitamine B2 19 à 119 µg, vitamine B6 8,5 à 45 µg, vitamine B12 0,02 à 0,36 µg, niacine 70 à 360 µg, acide folique 2,5 à 12 µg, acide pantothénique 96 à 478 µg, vitamine C 2,5 à 17 mg, biotine 0,4 à 2,4 µg, sodium 5 à 14 mg, potassium 14 à 43 mg, cuivre 8,5 à 29 µg, magnésium 1,2 à 3,6 mg, fer 0,1 à 0,36 mg, zinc 0,12 à 0,36 mg, manganèse 1,2 à 24 µg, calcium 12 à 35 mg, phosphore 6 à 24 mg, rapport entre calcium et phosphore 1:1 à 2:1, iode 2,5 à 14 µg, chlore 12 à 38 mg et sélénium 0,48 à 1,9 µg.

7. Composition selon l'une des revendications 1 à 4, **caractérisée en ce que** la composition comprend en outre une ou plusieurs des teneurs suivantes en oligo-éléments :
chrome 0,4 à 2,4 µg, molybdène 0,4 à 2,4 µg, choline 1,7 à 12 mg, inositol 1 à 9,5 mg, taurine ≤ 3 mg, L-carnitine ≥ 0,3 mg, acide docosahexaénoïque (% du total des acides gras) ≤ 0,5, et acide eicosatétraénoïque (% du total des acides gras) ≤ 1.

8. Composition selon l'une des revendications 1 à 4, **caractérisée en ce que** les nutriments et leur teneur par 100 kJ dans la composition comprennent : vitamine A 17,9 à 58,3 µgRE, vitamine D 0,25 à 0,75 µg, vitamine E ≥ 0,15 mg, vitamine K1 ≥ 1 µg, vitamine B1 ≥ 0,01 mg, vitamine B2 ≥ 0,01 mg, vitamine B6 ≥ 0,01 mg, vitamine B12 ≥ 0,04 µg, niacine ≥ 0,11 mg, acide folique ≥ 1 µg, acide pantothénique ≥ 0,07 mg, vitamine C ≥ 1,8 mg, biotine ≥ 0,4 µg, sodium 5 à 20 mg, potassium 18 à 69 mg, cuivre 7 à 35 µg, magnésium ≥ 1,4 mg, fer 0,25 à 0,5 mg, zinc 0,1 à 0,4 mg, manganèse 0,3 à 24 µg, calcium ≥ 17 mg, phosphore 8,3 à 46,2 mg, rapport entre calcium et phosphore 1:1 à 2:1, iode ≥ 1,4 µg, chlore ≤ 52 mg et sélénium 0,5 à 2,9 µg ;
les teneurs suivantes en oligo-éléments étant de préférence en outre ajoutées à la composition : chrome 0,4 à 5,7 µg, molybdène 1,2 à 5,7 µg, fluor ≤ 0,05 mg, choline 1,7 à 19,1 mg, inositol 1 à 9,5 mg, taurine ≤ 3,1 mg, L-carnitine ≥ 0,3 mg, acide docosahexaénoïque (% du total des acides gras) ≤ 0,5, acide eicosatétraénoïque (% du total des acides gras) ≤ 1, nucléotides ≥ 0,5 mg, et fibres alimentaires ≤ 0,7 g.

9. Composition selon l'une des revendications 1 à 4, **caractérisée en ce que** la composition est de la poudre.

10. Procédé de préparation de la composition pour le traitement de la diarrhée chez les nourrissons et les jeunes enfants selon l'une des revendications 1 à 9, comprenant :
(1) lavage du taro, épluchage, cuisson à la vapeur, échauffement pendant 5 à 30 minutes après l'ébullition de l'eau, et séchage à une température inférieure à 120 °C pour obtenir une poudre ;
(2) lavage de l'aliment antidiarrhéique, découpe en morceaux ou en granulés, ébullition dans de l'eau, poursuite de l'échauffement pendant 5 à 30 minutes après l'ébullition de l'eau, séchage et broyage ;
(3) mélange de la poudre de taro et de la poudre d'aliment antidiarrhéique selon le rapport, et stérilisation pour obtenir la composition ;
l'échauffement de l'étape (1) étant de préférence poursuivi pendant 10 à 15 minutes après l'ébullition de l'eau ; la température de séchage étant inférieure ou égale à 100 °C ; la température de séchage étant de manière plus préférentielle inférieure ou égale à 80 °C ; et
l'échauffement de l'étape (2) étant de préférence poursuivi pendant 15 à 20 minutes après l'ébullition de l'eau.

11. Composition alimentaire ou médicale selon l'une des revendications 1 à 9 pour l'utilisation dans le traitement de la diarrhée chez les nourrissons et les jeunes enfants.

12. Composition alimentaire ou médicale selon la revendication 11 pour l'utilisation dans le traitement de la diarrhée chez les nourrissons et les jeunes enfants, la diarrhée comprenant la diarrhée virale, la diarrhée bactérienne, la diarrhée allergique, la diarrhée due à une intolérance au lactose, la diarrhée d'origine médicamenteuse et la diarrhée causée par d'autres raisons ;
la composition étant de préférence administrée comme suit : 1 à 7 grammes à chaque prise pour les nourrissons âgés de 6 à 36 mois, 1 à 6 fois par jour ; et 2 à 10 grammes à chaque prise pour les enfants âgés de plus de 36 mois, 1 à 3 fois par jour.
